# EUROPEAN PATENT SPECIFICATION

(11) **EP 4 429 521 B1**
(45) Date of publication and mention of the grant of the patent: **30.09.2026**
(21) Application number: 22890515.4
(22) Date of filing: 12.10.2022
(51) Int. Cl.: A61G 12/00, A47K 10/18, A47F 5/00

(54) **WIRE FRAME HOLDING SYSTEM FOR PACKAGES**
DRAHTRAHMENHALTESYSTEM FÜR VERPACKUNGEN
SYSTÈME DE RETENUE EN FIL DE FER POUR COLIS

(30) Priority: 08.11.2021 SE 2151369
(43) Date of publication of application: 18.09.2024
(73) Proprietor: Svenska Good Medical AB, 448 43 Floda (SE)
(72) Inventor: GOOD, Anders, 448 34 Floda (SE); OSCARSSON, Per, 474 95 Hälleviksstrand (SE)
(74) Representative: Bergentall, Annika Maria
(86) International application number: PCT/SE2022/050925
(87) International publication number: WO 2023/080819

(56) References cited:
- WO-A1-2018/215281
- US-A- 1 894 597
- US-A- 3 927 768
- US-A- 4 867 318
- US-A1- 2013 062 300
- US-A1- 2016 029 786
- US-A1- 2016 278 518
- US-B1- 6 564 950

## Description

### TECHNICAL FIELD

The present invention relates to a holding unit for packages having the features of the first part of claim 1 and to a holding system having the features of claim 7.

### BACKGROUND

Different kinds of holding arrangements for packages, e.g., cardboard boxes, plastic containers or similar containing products, particularly disposable articles such as disposable gloves, masks etc., or refillable or disposable plastic containers or bottles holding liquids such as liquid soap, disinfectants etc., are known. Use of disposable articles is common not only within medical care where clean, hygienic articles are a requirement, but also within other fields where the requirements for hygiene and cleanliness are high, such as laboratories, within food industry and for production of products where the requirements are high on the products being clean, free from contamination, free from dust or similar just as well as refillable or disposable plastic containers or bottles holding disinfecting liquids.

Different problems are associated with the holding arrangements for holding such packages. It is a requirement that the products in the packages be easily accessible. The holding arrangements must admit packages to be held in a safe and stable manner, and such that products or articles held or contained therein will be easily accessible and the storing/holding must enable fulfilment of relevant requirements related to hygiene, otherwise in a serious case a consequence may be that bacteria are spread, and remaining articles are contaminated and/or that the package itself gets contaminated. It is also a requirement that the holding arrangements are easy to clean, are made of a material easy to keep hygienic standards. It is also a requirement that packages easily can be placed in the holding arrangement and that an empty package can be removed. The holding arrangement itself should also be easy to mount.

Many holding arrangements for packages which are adapted to be mounted on a wall are known; for holding packages with gloves or similar as well as for holding bottles with liquids. WO 0 074 530 shows one example of a holding arrangement for packages for gloves which is primarily intended for wall mounting. This holding arrangement comprises units for spring loading a package from above, to prevent a package from being displaced or falling out when an article is removed. It is preferably made of metal wires. WO 2014 148967 shows another arrangement for holding packages in which the packages are held such that the package openings will be located in an inclined plane to allow safe and easy removal of articles.

DE U 8624093 and SE 538 297 show examples of known arrangements for holding liquid dispensers.

In hospital environments, medical centers but also in laboratories etc. there is in general an extensive need for several different kinds of disposable articles held in packages, different kinds disposable or refillable containers which all need to be refilled and maintained. At a larger premise or at a hospital the number of articles of the same kind is also large, e.g. used in each room. All these articles and article holders need to be placed and removed, installed, be hygienic which calculated in time is an immense task and effort.

A disadvantage of known holding arrangements is that they are not flexible; they are generally secured to a wall by beans of screws or similar. If a different need arises, e.g., for holding more or less packages, a need of holding other packages, or reorganization, adaptation to other or new needs, the holding arrangement needs to be removed, screws or other fastening means removed leaving holes in the wall which in medical environments is not acceptable, new screw holes be made at a different location, etc.

In medical care environments such as hospitals the requirements on hygiene are very high, meaning not only that cleaning and disinfection should be performed frequently and after performing different kinds of tasks, it is also of extreme importance that it is easy to clean and disinfect, meaning that angles, wrinkles and corners where bacteria, viruses, fungi or other pathogens could collect and grow should be avoided to the largest possible extent and smooth materials are preferred.

It is also of importance that sharp corners, edges etc. are avoided to reduce the risk of damages.

US 4 867 318 A shows a storage rack for storing of items of various dimensional sizes such as video and audio recordings, which unit comprises a wire frame structure and an abutment member. US 2013/062300 A1 shows a rack system and a bracket wherein one or more arms or hooks can be inserted through apertures of a structural support means and protrude away from the support means and US 2016/029786 shows a shelving apparatus comprising a frame structure for receiving shelves.

Most known holding arrangements leave a lot to desire in particular as far as flexibility is concerned and also often suffer from disadvantages in the construction as to avoiding corners, angles, etc. rendering meeting of hygiene requirements difficult. As far as holding arrangements for packages, e.g. as shown in WO 0 074 530, the holding of packages is not stable enough and due to the construction, each time a package is placed therein or removed, the wire oval will get in contact with the wall on which the holding arrangement is mounted, and the wall will hence be exposed to wear and run the risk of being damaged which disadvantageous from a hygienic point of view and might require repair.

### SUMMARY OF THE INVENTION

It is therefore an object of the present invention to provide a solution to one or more of the above mentioned problems, and to provide a holding unit and a holding system through which one or more of the problems can be solved.

It is a particular object to provide a holding unit and a holding system respectively which is easy to mount and/or demount and which allows for easy adaptation to different needs and requirements.

A particular object is to provide a holding unit and a holding system which facilitates the maintaining of high demands on cleanliness and hygiene at the same time as it is easy to place and remove packages or containers.

A most particular object is to provide a holding unit and a holding system which allows a flexible use.

Still another particular object is to provide a holding unit which allows easy placement/removal of packages.

It is also an object to provide a holding unit and a holding system which is easy and cheap to fabricate, which is compact and, in addition thereto is durable.

Another particular object it to provide a holding system for which the holding functionality can be adapted.

A further particular object is to provide a holding unit and a holding system which is environment friendly, which shows advantages as far as storing and transportation are concerned, and which preferably is recyclable.

It is also a particular object of the invention to provide a holding system which can be made and adapted for use with varying needs concerning size, desired amount, type and location of packages to be stored in the holding system, available space for positioning of holding units etc.

Therefore, a holding unit as initially referred to is provided, which has the characterizing features of the characterizing part of claim 1.

Therefore, a holding system as initially referred to is provided which has the characterizing features of the characterizing part of claim 7.

Advantageous embodiments are given by the characteristic features of the dependent claims.

### BREIF DESCRIPTION OF THE DRAWINGS

The invention will in the following be further described, in a non-limiting manner, and with reference to accompanying drawings, in which:
Fig. 1 is a schematic view in perspective of a first embodiment according to the invention of a holding system comprising two holding units and a mounting rail,
Fig. 2 is a view in perspective of a mounting rail as schematically shown in Fig.1 but allowing reception of three holding units,
Fig. 2A is a front view of the mounting rail shown in Fig.2,
Fig. 2B is a side view of the mounting rail shown in Fig.2,
Fig. 3 is a view in perspective of a holding unit as in Fig.1 without a spring member,
Fig. 4 is a view in perspective of a spring member for a holding unit as in Figs. 1 and.3,
Fig. 4A is a side view of the spring member shown in Fig.4,
Fig. 4B is a view in perspective of the spring member shown in Fig.4 as seen from a rear side,
Fig. 5 is a schematic view in perspective of a holding unit as in Fig.3 with the spring member as in Fig.4,
Fig. 5A is a schematic front view of the holding unit in Fig.5,
Fig. 5B is a schematic top view of the holding unit in Fig.5,
Fig. 6 is a schematic view in perspective of a of a holding unit as in Fig.5 holding a package, and
Fig. 7 shows an alternative embodiment of a holding system comprising holding units with spring members as in Fig.4.

### DETAILED DESCRIPTION

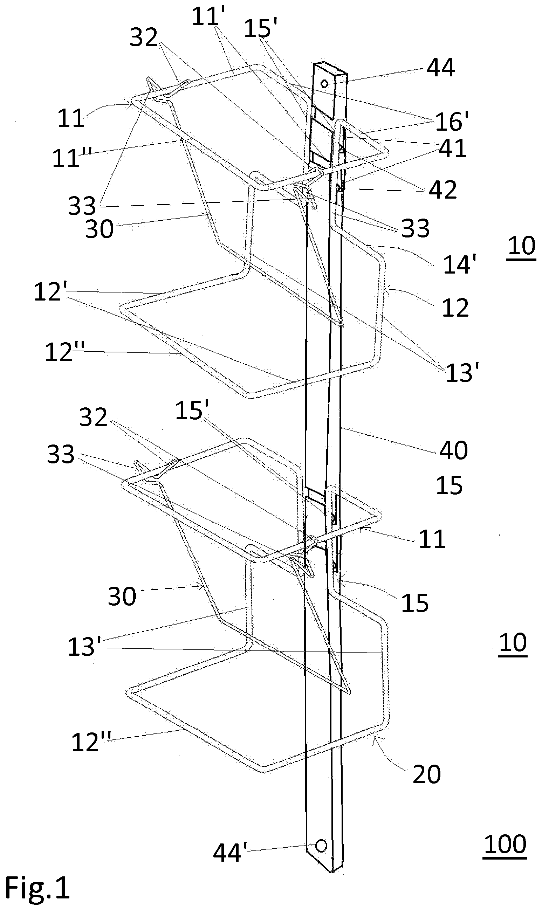

Fig. 1 is a view in perspective of a first embodiment of the present invention comprising a holding system 100 comprising a mounting rail 40 and two holding units 10,10 releasably mounted one above the other on the mounting rail 40 for holding packages for products, e.g. disposable articles.

The mounting rail 40 is here adapted for vertical mounting, e.g. on a wall by means of screws or similar (not shown) and comprises upper and lower openings 44,44' for reception of such screws.

Each holding unit 10 comprises a preferably wire-shaped frame structure 20 comprising two, an upper and a lower, frame elements sections 11, 12 which are interconnected through an interconnecting, or rear, section 15 as will be further described below.

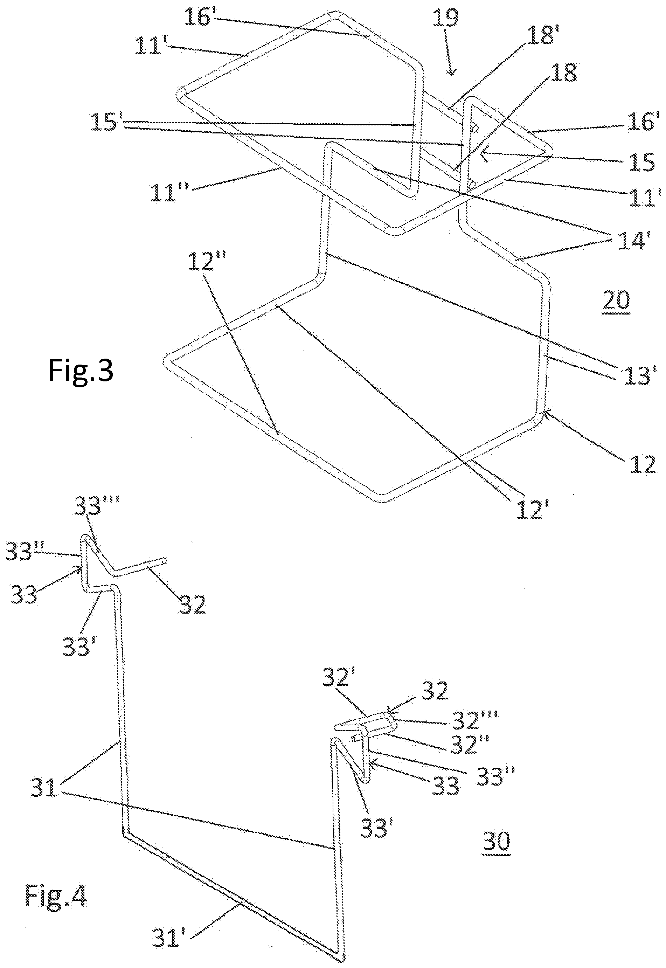

The lower frame element section 12 here has a substantially U-formed shape with two parallel lower leg portions 12',12' arranged to be disposed in a horizontal plane perpendicularly to the mounting rail 40 in a mounted state and a front leg interconnecting portion 12", allowing, in this embodiment, a package for gloves to rest thereon. The lower frame element section 12 front leg interconnecting portion 12" is located at a distance from the mounting rail 40 when the holding unit 10 is mounted on the mounting rail 40, and the two parallel lower leg portions 12',12', at the opposite ends, adjacent the mounting rail 40, in a mounted state when the holding units are arranged therein, show a respective bend of substantially 90° and extend in parallel upwards towards upper frame element section 11, here substantially halfway, each thus turning into a lower rear branch 13',13' of a lower rear section of the holding unit 10 frame structure 20. The lower rear branches 13',13' of the lower rear section extend in parallel, and, at a distance from the upper frame element section 11, e.g. half ways as referred to above, each form a bend of, here, substantially 90°, towards each other turning into, parallelly extending, upper rear branches 14',14' ( see Fig.3) of the lower frame element section 12. The upper rear branches 14',14', at a distance from each other corresponding to, or slightly exceeding, a width of the mounting rail 40 as will be further discussed below, form a bend of substantially 90° to form upper vertical rear branches 15',15' extending in parallel upwards until they, at a bend of substantially 90°, away from each other, turn into and form rear branches 16',16' of the upper frame element structure 11, thus forming an upper rear section 16 of the holding unit 10 frame structure 20.

The upper frame element section 11 here has a substantially U-formed shape with two parallel upper leg portions 11',11' and an upper front leg interconnecting portion 11" here of the same dimensions as, and parallel with, the two parallel lower leg portions 12',12' and front leg interconnecting portion 12" of the lower frame element section 12. The distance between the in parallel upper 11',11' and lower 12',12' leg portions of the upper and lower frame element sections 11,12 can be selected depending on the dimensions of a package to be held, but in general is between 100-200 mm, in advantageous embodiments between 130-180 mm, in some embodiments about 140-170 mm. Of course, other alternative distances are possible.

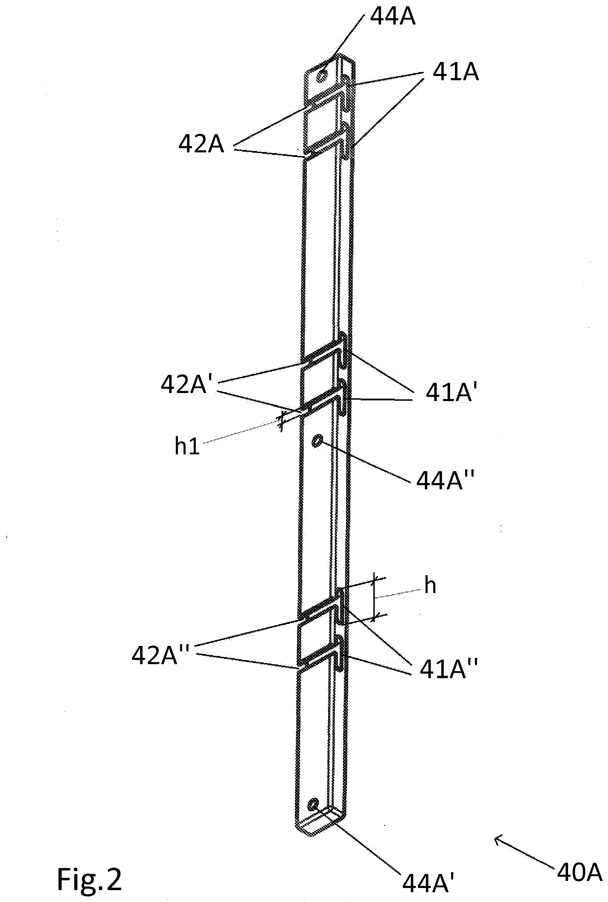

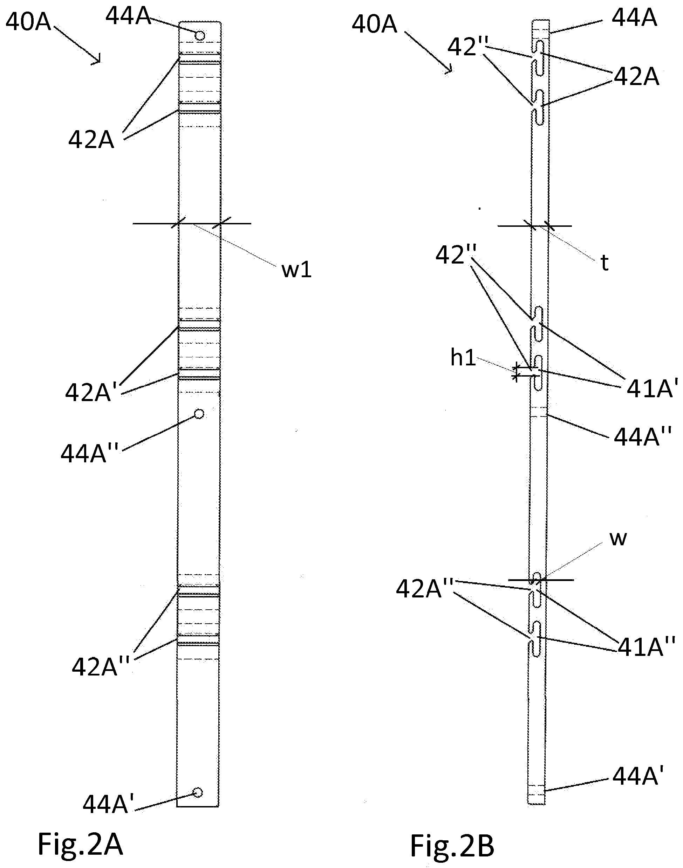

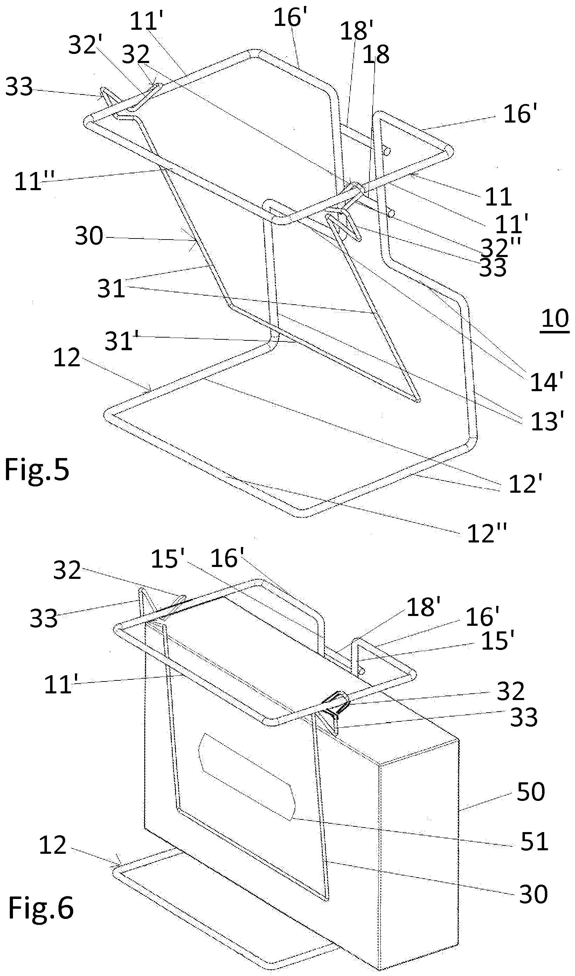

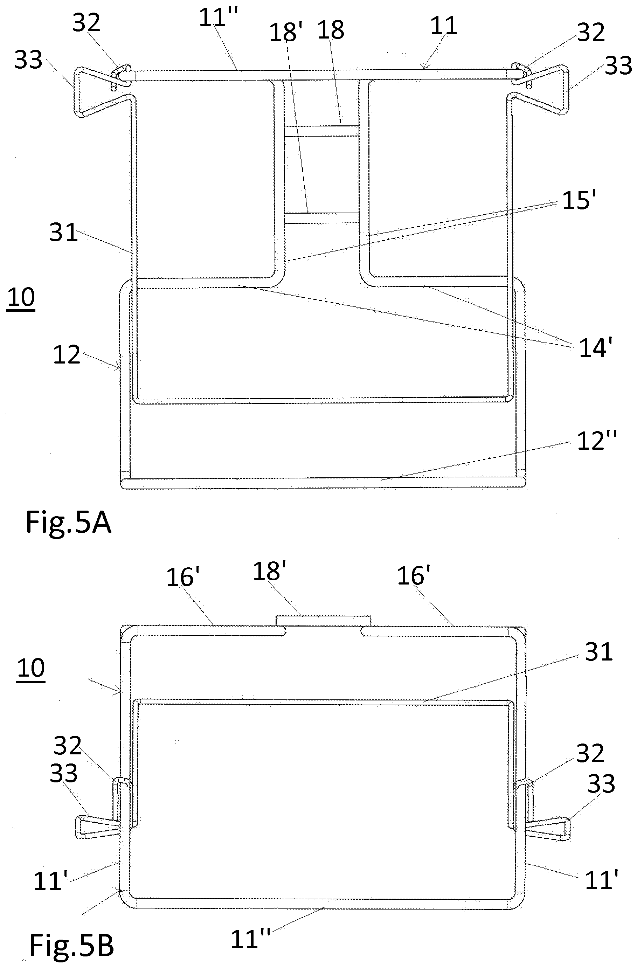

The (in a state for mounting the holding unit on a mounting rail 40) parallel, vertically disposed upper vertical branches 15',15' are interconnected by means of an upper 18' and a lower interconnecting rod 18 (not shown in Fig.1; cf. Figs.3,5) which are disposed in parallel at a distance from each other and form a connecting section 19, and which e.g. are soldered or welded onto the upper vertical branches 15',15'. Said upper and lower interconnecting rods 18',18 protrude slightly on a rear side of the holding unit 10 frame structure 20 and extend in parallel at a distance from each other (see Fig.3) allowing them to be received through receiving openings 42,42 and taken up in internal holding grooves 41,41 (see Fig.2) in the mounting rail 40. The rod holding grooves or accommodation spaces 41,41 are arranged in parallel across the width of the mounting rail 40, i.e. perpendicularly to the longitudinal extension of the mounting rail 40, and may have each a length of e.g. about 30 mm in one embodiment, although this is merely an exemplary figure; the width can be smaller as well as larger, e.g. between 15 and 80 mm as long as the lengths of the upper and lower interconnecting rods 18',18 is substantially the same, slightly exceeding it to allow for mounting of a holding unit frame structure 20 thereon.

The lengths of the interconnecting upper and lower rods 18',18 slightly exceed the width of the mounting rail 40 such that, when the holding unit 10 frame structure 20 is mounted on the mounting rail 40, the parallel, vertically disposed upper vertical branches 15', 15' will be located on either sides of the mounting rail 40 substantially without protruding, or without protruding more than to a minor extent, from a front plane formed by a front surface of the mounting rail 40 while the upper and lower interconnecting rods 18',18 are taken up in the internal rod holding grooves or accommodation spaces 41,41 (cf. Fig.2).

The height h1 (see Fig.2) of each respective receiving opening 42,42 exceeds the diameter or width of the upper 18' and a lower interconnecting rod 18 to allow for easy introduction thereof through the receiving openings 42,42 to be taken up in the internal holding grooves 41,41.

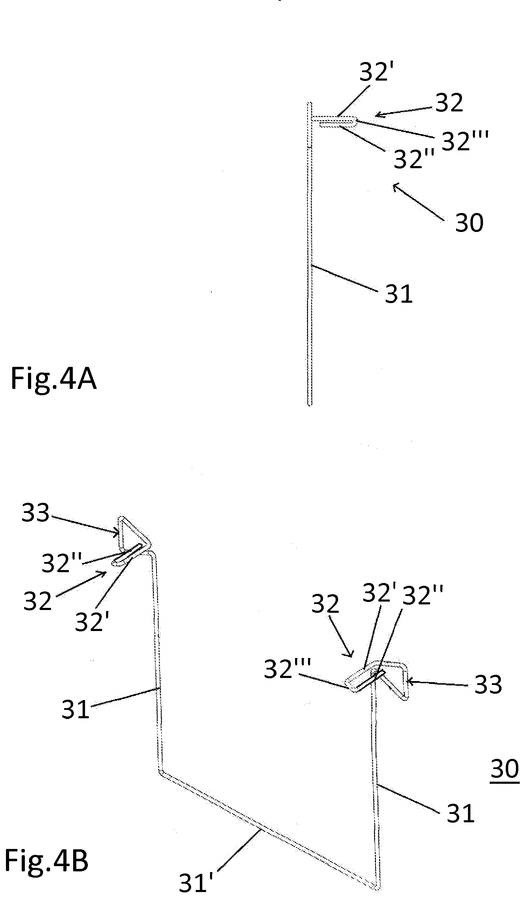

Each holding unit 10 also comprises a preferably, but not necessarily, releasably connectable spring member 30 which preferably comprises a U-shaped wire having a diameter slightly smaller than the diameter of the wire forming the frame structure 20. The spring member 30 is adapted to be fixedly or releasably connected to the two parallel upper leg portions 11',11' of the upper frame structure 20 of the holding unit 10 by means of connection portions 32,32 at ends of the U-shaped wire as will be further described below and with reference to Fig.4.

The spring member 30 (see Figs. 4,4A,4B) here comprises a U-shaped wire with two leg sections 31,31, an intermediate section 31' connecting the leg sections 31,31, two connection portions 32,32 disposed at the leg section 31,31 ends opposite to the intermediate section 31' allowing connection to or mounting on the two parallel upper leg portions 11',11' of the upper frame element section 11 of the frame structure 20, and two actuation members 33,33.

Each connection portion 32 and each actuation member 33 (further illustrated in Fig.4) are formed at an opposed end of a leg section 31 which forms a bend to provide said actuation member 33 which protrudes outwardly, away, in a same plane as a plane formed by the leg sections 31,31 and the intermediate section 31', from the leg section 31 where it is disposed; the two actuation members 33,33 hence protruding in opposite directions.

Each actuation member 33 ( see Fig.4) in the shown embodiment comprises an outwardly flaring section of a wire formed by the end of each leg section 31 forming a first bend (between 90° and 180°, preferably about 100°-135°, in the plane of the spring member 30, forming a first actuation member section 33', at a distance therefrom of e.g. 10-25 mm a second bend of e.g. more than 90°, to form a second actuation member section 33", here disposed to extend in parallel with the spring member leg sections 31,31, and a third bend, e.g. of a same angle as the second bend but with opposite sign, forming a third actuation member section 33‴ flaring inwardly towards the first bend and within the same plane but being somewhat shorter than the second actuation member section 33‴ such that a distance is left between the first and a subsequent fourth bend of the wire section forming an actuation member 33 shaped and sized to allow comfortable pushing and pulling. In advantageous embodiments the actuation members protrude outwardly in the same plane a distance from the respective leg section 31 of about 15-30 mm.

It should be clear that the wire section forming an actuation member also could be shaped in other, different manners and e.g. have the shape of an eyelet, be semicircular or oval shaped etc., or even be provided as a plate or similar connected to the wire section, such that it can be used as an actuation member and the two actuation members of the spring member 30 extending outwardly in opposite directions allowing simultaneous pulling and pushing or gripping to move the spring member 30 as will be further described below.

At the end of each third actuation member section 33‴ the wire shaped element forms a bend (the fourth bend) of substantially 90° with respect to the respective spring member leg sections 31 inwardly, perpendicularly to the plane formed by the spring member 30 wire structure, to form a U-shaped connection portion 32. A free leg 32" of the U-shaped connection portion 32 is somewhat shorter than the other leg 32' of the U-shaped connection portion 32 connecting to the actuation member section 33‴, and the U-shaped connection portion 32 is so arranged that a plane formed by the parallel legs 32',32'' and interconnecting leg 32‴ of the connection portion 32 forms an angle of about +45°/-45° respectively with a vertical plane perpendicular to the plane formed by the two spring member leg sections 31,31 and intermediate section 31'.

The connection portions 32, 32 are arranged to allow the spring member 30 to slide on the two parallel upper leg portions 11',11' of the upper frame element section 11, the two parallel upper leg portions 11',11' being taken up between the inner leg and the free leg 32',32'' of a respective connection portion 32,32, and, the spring member 30 will, in an unloaded position resting on the two parallel leg portions 11',11', pend downwards, inwardly towards the rear section 15 of the wire shaped frame structure 20.

Through simultaneously pushing or pulling the actuation members 33,33 in a same direction, the spring member 30 can be freely moved, sliding, between a rear position close to, but at a distance from the rear section 15 (the movement being restricted through the angular disposition of the respective connection portion 30 including the fourth bend of the actuation member 33 on the leg portion 11') and a front position adjacent front leg interconnecting portion 11" through the U-shaped connection portions 32,32 sliding on the two parallel upper leg portions 11',11'

When the spring member 30 is moved to the front position, i.e. to a position at a slight distance therefrom, a package 50, e.g. holding gloves, can be introduced form any side, in parallel with a plane formed by the rear section 15, with its bottom sliding on the two parallel lower leg portions 12',12' of the lower frame element section 12 during introduction, to rest thereon in a holding state, the package opening being located between the spring member 30 and the rear section 15 (cf. Fig. 6 showing a package 50 held in a holding unit 10).

Once a package 50 has been introduced and is located in an appropriate position, e.g. leaving an access opening 51 free for access and the ends of the package protruding a similar distance on either side of the holding unit 10, in order to ensure that the package 50 will be held in place and not fall out, the actuating members 32,32 are simultaneously pushed towards the rear section 15, the intermediate section 31' of the spring member 30 first reaching the package 50, and upon pushing further on the actuating members 32,32, the connection portions 32,32 will be exposed to a rotational movement and biased at the same time as the friction increases between the third actuation member sections 33‴, 33''', the inner sides of the inner legs 32',32' of the connection portions 32,32, an upper side of the actuation member section 33‴ adjacent the fourth bend disposed below the upper leg portion 11', an inner side of interconnecting leg 32‴ of the angled connection member 32 which is located above the upper leg portion 11', and the respective parallel upper leg portions 11',11' of the upper frame element section 11.

A package will thus be held in place through a combination of a torsional force and a frictional force between the connection portions 32,32 (including upper side of the actuation member section 33‴ adjacent the fourth bend) of the spring member 30 and the parallel leg portions 11',11';11',11' of the upper frame element section 11. To remove a package, the actuating members 33,33 are simply gripped and pulled simultaneously outwardly, in a direction pointing away from the wall or the rear section 15.

Through the combination of a torsional force and a frictional force the spring member 30 will be held in place and prevented from being unintentionally displaced.

Through the free U-leg 32"of the U-shaped connection portion 32 also being somewhat shorter than the other parallel leg 32' thereof, the spring member 30 can be releasably arranged on the upper frame section element 11 wire shaped frame structure 20 of the holding unit 10.

Fig.2 is a view of a mounting rail 40A similar to the mounting rail 40 of Fig.1 with the difference that it is adapted to allow reception of three holding units as shown in Fig.1. The mounting rail 40A comprises upper and lower openings 44A,44A' and in addition thereto an intermediate opening 44A" located for reception of screws or similar for secure mounting on a wall.

The mounting rail 40A is provided with holding grooves or accommodation spaces 41A,41A, 41A',41A', 41A",41A" which are arranged pairwise and perpendicularly to the longitudinal extension of the mounting rail 40A, each pair allowing reception of a holding unit (not shown) e.g. as described with reference to Fig.1, through respective receiving openings 42A,42A, 42A',42A',42A",42A" on a front side of the mounting rail 40A. Each receiving opening 42A,42A, 42A',42A',42A",42A" is located to provides access to each a said holding groove or accommodation space 41A,41A,41A',41A',41A",41A". Each holding groove 41A,41A, 41A',41A',41A",41A" has a longitudinal dimension or groove height h, taken in the longitudinal direction of the mounting rail 40A, exceeding the width of the receiving opening 42A,42A, 42A',42A',42A",42A" and extending at least downwards below the receiving opening 42A,42A, 42A',42A',42A",42A" within the mounting rail 40A in a direction towards a lower end of the mounting rail 40A in a mounted state on a wall or similar; in this embodiment also upwards from the receiving opening 42A,42A, 42A',42A',42A",42A".

Each holding groove or accommodation space 41A,41A, 41A',41A',41A",41A" has a groove width w (see Fig.2B) somewhat exceeding the diameter of a holding unit 10 upper 18' and lower interconnecting rod 18 as discussed with reference to Fig.1.

In a particular embodiment the mounting rail 40A has a width w1 (see Fig.2A) of about 30 mm, the figure merely being given for exemplifying one specific embodiment; the width can be smaller as well as larger e.g. between 15 and 80 mm as long as the length of the upper and lower interconnecting rods 18',18 of a wire-shaped frame structure 20 is substantially the same, but slightly exceeding it, to allow for mounting of a holding unit 10 (see Fig.1). The mounting rail may have a thickness t (see Fig.2B) of between 8 -1.5 mm, but also other dimensions, smaller as well as larger, are possible.

The height h1 (see Fig.2B) of each respective receiving opening 42A,42A, 42A',42A', 42A",42A" somewhat exceeds the diameter of the upper and a lower interconnecting rods 18',18 to allow the upper and a lower interconnecting rods 18',18 to be easily introduced through the receiving openings. When received through the receiving openings, the upper and a lower interconnecting rods 18',18 are moved vertically downwards within the internal holding grooves 41A,41A, 41A',41A', 41A'',41A'' until they reach bottom surfaces thereof. A holding unit 10 will thus be securely held in place until being lifted up and the upper and a lower interconnecting rods 18',18 being withdrawn through the respective receiving openings, e.g. for cleaning purposes or for replacement through another holding unit adapted to hold another type or dimension of packages or for any other reason.

In the shown embodiment, to which the inventive concept by no means is limited, each receiving opening 42A,42A, 42A',42A', 42A",42A" has a lower edge which is slightly slanted outwards so as to facilitate insertion of the interconnecting rod 18,18' and an upper edge which is slanted slightly inwards, upwards. In alternative embodiments only the lower, only the upper or none of the lower and upper edges are slanted.

Fig.2A is a schematic front view of the mounting rail 40A shown in Fig.2 wherein dashed lines indicate the internal holding grooves or accommodation spaces 41A,41A, 41A',41A', 41A",41A". Fig.2A will not be further discussed here since all elements and features have already been described with reference to Fig.2.

Fig.2B is a schematic side view of the mounting rail 40A shown in Fig.2 indicating the thickness t of the mounting rail 40A; the height h1 of the receiving openings and the width w of the holding grooves. In other respects Fig.2B will not be further discussed since all elements have already been described with reference to Fig.2.

It is an advantage that a mounting rail 40,40A as described herein is easy to fabricate and easy to mount. It can be made in different lengths; a rail being simply cut into the desired length and the grooves and holes being provided through milling. In an advantageous embodiment it is made of Al, more particularly of anodized Al, although other materials also can be used.

It is also an advantage that such a mounting rail is recyclable.

Fig. 3 is an enlarged view of a wire shaped frame structure 20 of a holding unit 10 as described with reference to Fig.1, without any spring member mounted thereto. The rear section 15 comprising the lower rear section formed by lower rear branches 13',13' and connecting section 19 formed by upper 18' and lower interconnecting rods 18 in parallel at a distance from each other which e.g. are soldered or welded onto the upper vertical branches 15',15' is more clearly illustrated. In other respects the wire shaped frame structure 20 has been thoroughly described with reference to Fig.1 and will therefore not be further described here.

Fig.4 is an enlarged view in perspective from the front of a spring member 30 as described with reference to Fig.1 for mounting on the upper frame element sections 11',11' of the wire shaped frame structure 20 shown in Fig.3. Due to the distance between the respective free 32"of the U-shaped connection portion 32, which is somewhat shorter than the respective parallel inner leg 32' of the U-shaped connection portion 32, spring member 30 can be mounted on the upper frame section element 11 of the wire shaped frame structure 20, although in other implementations the spring member 30 is not removable from the upper frame element section 11. The spring member 30 will not be further described with reference to Fig 4 since it has been described in detail above with reference to Fig.1, also referring to Fig.4.

Fig.4A is a schematic side view of the spring member 30 of Fig 4 shown merely for illustrative purposes showing the free leg 32" of the U-shaped connection portion 32, which is somewhat shorter than the inner leg 32' of the U-shaped connection portion 32.

Fig.4B is an enlarged view in perspective of the spring member 30 in Fig.4 seen from the opposite side, i.e. in perspective from a rear side, shown merely for the purposes of clearly illustrating the spring member 30 and which will not be further discussed herein for all elements and features already having been discussed above.

Fig.5 is an enlarged view of a holding unit 10 comprising a wire shaped frame structure 20 and a frame member 30 as shown in Fig.1, all features of which already having been discussed with reference to Fig 1.

Fig.6 shows the holding unit 10 of Fig 5 holding a package 50, e.g. containing disposable gloves, having a front opening 51 through which access is provided to the content in the package.

The spring member 30 is now in a holding position in which the package 50 is clamped between the spring member 30 and the rear section 15 of the holding unit 10.

For introduction of the package 50, the spring member 30 has been moved through simultaneously gripping or pulling the actuation members 33,33 away from the rear section 15, towards the front on the upper frame element sections 11',11' on which the connection portions 32,32 can slide freely in an unloaded state; pending downwardly in an angled position as shown in Fig. 5. A package can then be introduced into/removed from the holding unit 10 from any side through a transverse movement sliding on the lower frame element 12 leg portions 12',12' perpendicularly to a longitudinal extension of the leg portions 12',12' such that the bottom of the package 50, opposite to a front or upper side provided with opening 51 is in sliding contact with the rear section 15.

For securing the package 50 in place a user pushes the spring member 30 towards the rear section by simultaneously and applying substantially the same force to the two actuation members 33,33 whereupon the spring member 30 will be urged towards the package by means of a combination of torsion and friction between the connection portions 32,32 and the upper frame element sections 11',11' as described with reference to Figs.1,3,4 above, and when the leg sections 31,31 and the intermediate section 31' of the spring member 30 are in direct contact with the package 50 throughout their extension, the package 50 will be held safely in place, the spring member 30 extending in a plane substantially parallel to a plane formed by the rear section 15.

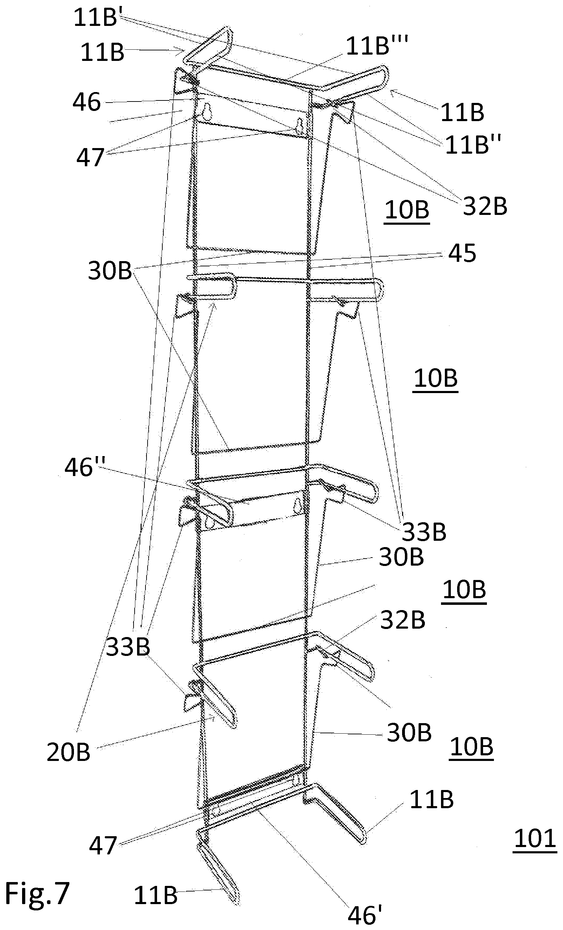

Fig.7 shoes another embodiment of a holding system 101 according to the invention in which a number of holding units 10B, here four holding units 10B, are arranged vertically one above another. Each holding unit 10B is fixedly mounted, e.g. through welding or soldering, on a mounting rail arrangement 40B formed by two parallel vertically extending (in a mounted state of the holding system 101 on a wall or similar) branches 45,45 which are interconnected by means of at least an upper and a lower connection member 46,46',46", in the shown embodiment connection plates soldered or welded onto upper, lower and intermediate sections of the parallel branches 45,45. In the shown embodiment the connection plates 46,46',46'' are provided with pairwise disposed holes 47,47, key holes or similar, for screws, hooks or similar for mounting onto a wall. The spring members 30B of each holding unit 10B,10B,10B are similar to the spring members 30 described with reference to Figs.4,4A and 4B and will therefore not be further discussed here. Also, the functioning of the spring member 30B in combination with upper frame element section 11',11' (here corresponding to lower branch portions 11B",11B" as will be further described below) is similar to that described with reference to the preceding embodiments and will therefore not be further discussed here.

The holding unit 10B in this embodiment only comprises an upper frame element section 11B and a spring member 10B. The upper frame element section 11B comprises two parallel, an upper and a lower, branch portions 11B',11B" protruding from, and perpendicularly to, each a said of the two parallel vertically extending (in a mounted state of the holding system 101 on a wall or similar) branches 45,45 in a forward direction away from a wall or similar.

The upper branch portions 11B'' are interconnected by means of an intermediate rear branch 11B‴ through the wire structure forming the upper branch portion 11B',11B' at a respective rear end forming a 90° bend, the intermediate rear branch 11B‴ hence extending perpendicularly to, and being welded or soldered onto, the two parallel vertically extending (in a mounted state of the holding system 101 on a wall or similar) branches 45,45 in a rear plane. The length of the intermediate rear branch 11B‴ exceeds the distance between the branches 45,45 such that the upper branch portion 11B',11B' protrude perpendicularly to the branches 45,45, on opposite sides thereof.

Each upper branch portion 11B',11B', made of a wire structure, at a distance from a respective rail 45 is bent substantially 180° to extend in parallel below the upper branch portion 11B' forming or turning into a lower branch portion 11B" of the upper frame element section at a distance therefrom to the rail 45 to which it secured e.g. by welding or soldering.

Thus, the cooperation between the spring member 30B and lower branch portion 11B'' of the upper frame element section corresponds to that described earlier with reference to the preceding figures of the present application between spring member 30 and upper frame element sections 11',11'.

Each upper branch portion 11B',11B' of a holding unit 10B serves substantially as a lower frame element section 12 as described earlier in the application in that a package may be received thereon, and hence serves as a receiving lower section of another holding unit 10B disposed vertically above the holding unit of which it here is defined as forming a part.

An advantage of the present invention is that, through the inventive concept, a holding system is provided wherein, when mounted onto a wall, the movement of the spring member 30, to allow placement of, or removal of a package, can be moved from a release position to a clamping position without any rear portion protruding outwardly during the movement of for example the spring member and possibly damaging a wall on which the holding system is mounted, which is of particular importance for example in medical or laboratory settings, such as in hospitals, where the requirements as to a high hygiene are extremely important.

It is also an advantage that, in some advantageous embodiments, the spring members can be removed and easily cleaned, e.g. in a dish washer, and that, in addition, the holding units can be removed and cleaned or sterilized in an easy manner, e.g. in a medical autoclave, in a dish washer or similar.

Another advantage is that the number of protruding edges, wrinkles etc. are few compared to for known holding arrangements minimizing the risk for collection of bacteria, viruses, fungi or other pathogens could collect and grow.

The holding units and the spring members are advantageously made of a wire shaped material of metal, particularly preferably stainless steel, acid proof steel or spring steel but also other materials are possible to use.

Through the use of wire elements (the flexibility can also be provided for through elements which are formed in different manners, for example sheet spring steel or some other band shaped element) the spring member will be is flexible and resilient, which means that the holding element will be adaptable to different thicknesses and irregularities of packages. By using spring steel the flexibility can also be further enhanced.

Since there are no side sections on the holding units, there is a high flexibility as far as the width of the packages to be held therein is concerned.

It is also an advantage that a concept of a holding system and a holding unit respectively is provided which is very flexible and which simply can be manufactured for different needs and depending on available space, for vertical mounting of holding units in any desired number.

If the holding units are made of stainless steel, they are to 100% recyclable which provides an environmental advantage.

The holding units, the shape of the actuation members, the shapes of the spring member legs, any mounting rail arrangement can be varied in a number of different manners. It should be clear that different features in different embodiments freely can be combined and varied in any desired manner within the scope of the invention and the invention is not limited to the particularly illustrated embodiments, but it can be freely varied within the scope of the appended claims. If holding units are detachably arranged, it is also possible to, in one and the same holding system, use holding units for different dimensions, or even different holding units for different types of packages, for example for packages for disposable gloves and packages for masks, aprons or other disposable articles which for example are kept in packages of different forms and/or sizes.

It is also possible to use a mounting rail as described with reference to Fig.1, Figs 2-2B also for other types of holding units adapted to hold packages or containers of any desired kind and which are provided with a rear section adapted for cooperation with holding grooves and receiving openings as described above.

It is also possible to use detachable holding units as described with reference to Fig.3 for such a mounting rail using any other type of spring member arrangement, such a spring member may also disposed in any other appropriate manner, for securing a package or similar.

## Claims

1. A holding unit (10;10B), comprising a wire frame structure (20;20B) and a spring member (30;30B) made of wire, adapted for reception and retaining of a package (50) so that access is admitted to products or articles in the package (50),
**characterized in**
**that** it comprises an upper frame element section (11;11B) comprising two parallel upper leg portions (11',11';11B",11B") protruding substantially horizontally and perpendicularly from a rear section (15) of the holding unit, that the spring member (30;30B) is substantially U-leg-shaped and comprises an actuation member (33,33;33B,33B) and a connection portion (32,32;32B,32B) at both ends of parallel legs (31,31) of said spring member (30;30B), that the connection portions (32,32;32B,32B) of the spring member (30;30B) are slidably mounted on the parallel upper leg portions (11',11';11B",11B") such that the spring member (30;30B) can be moved between a rear position close to the rear section (15) of the holding unit (10;10B) and a front position adjacent outer end portions of the parallel upper leg portions (11',11';11B",11B") by the connection portions (32,32;32B,32B) sliding on the two parallel upper leg portions (11',11';11B",11B") through simultaneous application of a pushing/pulling force on each actuation member (33,33;33B,33B), and in that the connection portions (32,32;32B,32B) are so shaped and arranged on the parallel upper leg portions (11',11';11B",11B") that, in an unloaded state, the spring member (30;30B) mounted by means of the connection portions (32,32;32B,32B) will hang such that a lower intermediate section (31') connecting the parallel legs (31,31) of said spring member (30;30B) will be located closer to the rear section (15) of the holding unit (10;10B) than the connection portions (32,32;32B,32B), whereas in a loaded state, e.g. with a package disposed between rear section (15) of the holding unit (10;10B) and the spring member (30;30B), the actuation members (33,33;33B,33B) pushed towards the rear section (15) of the holding unit (10;10B), urging the spring member (30;30B) to assume a vertical position parallel to the rear section (15) of the holding unit (10;10B) in a position for receiving a package, through a combination of a frictional and a torsional force being exerted between the connection portions (32,32;32B,32B) the and parallel upper leg portions (11',11';11B",11B"), such that the spring member (30;30B) will apply a force along the parallel legs (31,31) allowing e.g. a package (50) to be clamped between the spring member (30;30B) and the rear section of the holding unit (10;10B).

2. A holding unit (10;10B) according to claim 1,
**characterized in**
**that** the, or each, spring member (30,30;30;30B,...,30B) is adapted to be fixedly or releasably connected to the two parallel upper leg portions (11',11';11B",..,11B"), the two actuation members (33,33) and the two connection portions (32,32) being disposed at the leg section (31,31) ends opposite to the intermediate section (31'), that the actuation members (33,33) of a spring member (30,30;30;30B,...,30B) flare outwardly in opposite directions facing away from the holding unit (10,10;10B,...,10B) in opposite directions, each comprising an outwardly protruding bent wire portion extending in the same plane as a plane formed by the spring member leg sections (31,31) and intermediate section (31'), wherein an upper leg forming a third actuation member section (33‴) at an inner, upper, end of each third actuation member forms a bend of substantially 90° with respect to the respective spring member leg section (31,31), perpendicularly to the plane formed by the spring member (30,30;30;30B,...,30B) wire structure, to form said connection portion (32,32) having a form of a U with a free leg (32",32") somewhat shorter than a parallel, inner, leg (32',32') of the U-shaped connection portion (32,32) connecting to the third actuation member section (33‴,33‴), said U-shaped connection portion (32,32) being so arranged that a plane formed by the parallel legs (32',32") of the connection portion (32,32) forms an angle of between +/-30-45° respectively with a vertical plane perpendicular to the plane formed by the two spring member leg sections (31,31) and intermediate section (31').

3. A holding unit (10;10B) according to any one of claims 1 or 2,
**characterized in**
**that** the spring member (30,30;30B,..,30B) comprises a wire element of metal, for example of stainless steel or spring steel or of another material.

4. A holding unit (10;10B) according to claim any one of claims 1-3,
**characterized in**
**that** the wire-shaped frame structure (20,20;20B,...,20B), or at least the upper frame element sections (11,11;11B,..,11B), is/are made of wire-shaped metal, e.g. stainless steel, and in that the diameter of the wire element forming the spring member (30,30;30B,..,30B) is smaller than the diameter of the wire forming the frame structure (20,20;20B,...,20B) or at least the upper frame element sections (11,11;11B,..,11B).

5. A holding unit (10;10B) according to any one of claims 1-4,
**characterized in**
**that** the spring member (30,30;30B,..,30B) is adapted to be arranged on the two parallel upper leg portions (11',11';11B",..,11B") such that each parallel upper leg portion (11',11'; 11B",..,11B") is taken up between the legs (32',32") of the connection portion (32,32), an upper side of an upper actuation member section (33‴) adjacent the bend where it is turns into the inner leg (32') of the connection portion (32,32) and a lower side connection portion (32) intermediate portion (32‴), and is in contact with a central portion of the inner leg (32') of the connection portion (32,32) and the upper side of the upper actuation member section (33‴) adjacent the bend and the lower side of the intermediate portion (32‴) of the connection portion (32).

6. A holding unit (10) according to any one of claims 1-5,
**characterized in**
**that** the wire frame structure (20,20) comprises a lower frame element section (12), that the lower frame element section (12) has a substantially U-formed shape with two parallel lower leg portions (12',12') and a front leg interconnecting portion (12") arranged to be disposed in a horizontal plane perpendicularly to the mounting rail arrangement (40) in a mounted state in a plane parallel to the upper frame element section (11) and serving as a support for a package to be held, that the wire frame structure (20,20) further comprises a rear section (15) comprising two parallel disposed upper vertical branches (15',15') interconnected by means of an upper and a lower interconnecting rod (18',18) which are disposed in parallel at a distance from each other and which e.g. are soldered or welded onto the upper vertical branches (15',15') on a rear side thereof and protruding a slight distance therefrom forming a connection section (19) for connection to the mounting rail arrangement (40;40A).

7. A holding system (100;101) for packages for products, for example disposable articles, comprising a mounting rail arrangement (40;40A;40B) for vertical mounting on a vertical surface, **characterized in**
**that** it comprises one or more holding units (10,10;10B,...,10B) according to any one of claims 1-6 fixed or detachably mounted to the mounting rail arrangement (40;40A;40B),
wherein the two parallel upper leg portions (11',11';11B",..,11B") of the, or each, holding unit (10,10;10B,...,10B) protrude substantially horizontally and perpendicularly from the mounting rail arrangement (40;40A;40B), wherein the spring member(s) (30,30;30;30B,...,30B) of the, or each, holding unit (10;10B) can be moved between a rear position close to a vertical plane of extension of the mounting rail arrangement (40;40A;40B) and the front position adjacent outer end portions of the parallel upper leg portions (11',11'; 11B",..,11B"), and, in the unloaded state, the spring member (30,30;30;30B,...,30B) of the, or each holding unit (10;10B) will hang such that the lower intermediate section (31) connecting the parallel legs (31,31) of said spring member (30,30;30;30B,...,30B) will be located closer to the vertical plane of extension of the mounting rail arrangement (40;40A;40B) than the connection portions (32,32;32B,..,32B), whereas in the loaded state, the spring member(s) (30,30;30;30B,...,30B) are urged towards the vertical plane of extension of the mounting rail arrangement (40;40A;40B through said combination of a frictional and a torsional force exerted between the connection portions (32,32;32B,..,32B) and the parallel upper leg portions (11',11'; 11B",..,11B"), allowing e.g. a package (50) to be clamped between the spring member (30,30;30;30B,...,30B) of a respective holding unit (10,10;10B,...,10B) and the mounting rail arrangement (40;40A;40B) or the rear section of the respective holding unit (10,10;10B,...,10B).

8. A holding system (100) according to claim 7,
**characterized in**
**that** mounting rail arrangement comprises a mounting rail (40;40A) which comprises internal holding grooves or accommodation spaces (41A,41A,41A',41A',41A",41A") which are arranged pairwise and perpendicularly to the longitudinal extension of the mounting rail (40:40A), each pair of internal holding grooves or accommodation spaces (41A,41A,41A',41A',41A",41A") allowing reception of a holding unit (10,10) through reception of the upper and lower interconnecting rods (18',18) at a rear section of the wire-shaped frame structure (20,20) through respective receiving openings (42A,42A, 42A',42A',42A",42A") on a front side of the mounting rail (40;40A), the mounting rail (40;40A) further comprising mounting elements or mounting holes (44,44';44A,44A',44A") or similar for mounting on a wall.

9. A holding system (100) according to claim 8,
**characterized in**
**that** each holding groove or accommodation space (41A,41A,41A',41A',41A",41A") has a height (h) exceeding a height (h1) of a corresponding receiving opening (42A,42A, 42A',42A',42A",42A") and in that a lower end portion of the internal groove holding groove or accommodation space (41A,41A,41A',41A' ,41A",41A") is located below a lower edge of the corresponding receiving opening (42A,42A, 42A',42A',42A",42A") in a vertically disposed state of the mounting rail (40;40A) allowing a mounted holding unit to be held in place through the upper and lower interconnecting rods (18',18) being taken up within the internal groove holding groove or accommodation space (41A,41A,41A',41A' ,41A",41A") in contact with bottom portions thereof.

10. A holding system (100) according to any one of claims 7-9,
**characterzized in**
that the or each holding unit (10,10) is detachably mountable to the mounting rail (40).

11. A holding system (100) according to any one of claims 7-10,
**characterized in**
**that** the mounting rail (40) is made of Al, more particularly of anodized Al.

12. A holding system (101) according to claim 7,
**characterized in**
**that** the mounting rail arrangement (40B) comprises two parallel, in a mounted state of the mounting rail arrangement (40B) vertically extending branches (45,45) which are interconnected by means of at least an upper and a lower connection member (46,46',46'') for mounting onto a wall or similar, a number of holding units (10B,10B,10B,10B) arranged vertically one above another, each holding unit (10B,10B,10B,10B) being fixedly mounted, e.g. through welding or soldering, onto the vertically extending branches (45,45) of the mounting rail arrangement (40B), each holding unit (10B,10B,10B,10B) comprising an upper frame element section (11B,11B,11B,11B) comprising two parallel, an upper and a lower, U-shaped branch portions (11B',11B") protruding from, and perpendicularly to, each a said of the two parallel vertical branches (45,45) in a forward direction, said upper branch portions (11B",11B") being interconnected by means of an intermediate rear branch (11B‴), the intermediate rear branch (11B‴) extending perpendicularly to, and being welded or soldered onto, the two parallel vertically extending branches (45,45) in a rear plane, the upper branch portions (11B',11B') of a holding unit (10B), having a holding unit arranged above it, serving as a support plane, e.g. for a package, of the holding unit arranged above it, and the spring member (30B) being arranged on the lower branch portions (11B",11B"), each upper frame element section (11B) being made of a bent wire structure.

## Patentansprüche

1. Halteeinheit (10; 10B), umfassend eine Drahtgestellstruktur (20; 20B) und ein Federelement (30; 30B) aus Draht, die zur Aufnahme und zum Halten einer Packung (50) ausgebildet sind, sodass der Zugang zu Produkten oder Artikeln in der Packung (50) ermöglicht wird,
**dadurch gekennzeichnet,**
**dass** sie einen oberen Rahmenelementabschnitt (11; 11B) umfasst, der zwei parallele obere Schenkelabschnitte (11', 11'; 11B", 11B") aufweist, die im Wesentlichen horizontal und senkrecht von einem hinteren Abschnitt (15) der Halteeinheit abstehen, dass das Federelement (30; 30B) im Wesentlichen U-Schenkelförmig ausgebildet ist und ein Betätigungselement (33, 33; 33B, 33B) sowie einen Verbindungsabschnitt (32, 32; 32B, 32B) an beiden Enden paralleler Schenkel (31, 31) des Federelements (30; 30B) umfasst, dass die Verbindungsabschnitte (32, 32; 32B, 32B) des Federelements (30; 30B) verschiebbar auf den parallelen oberen Schenkelabschnitten (11', 11'; 11B", 11B") angeordnet sind, sodass das Federelement (30; 30B) durch gleichzeitiges Aufbringen einer Schub /Zugkraft auf jedes Betätigungselement (33, 33; 33B, 33B) zwischen einer hinteren Position nahe dem hinteren Abschnitt (15) der Halteeinheit (10; 10B) und einer vorderen Position nahe äußeren Endabschnitten der parallelen oberen Schenkelabschnitte (11', 11'; 11B", 11B") bewegt werden kann, indem die Verbindungsabschnitte (32, 32; 32B, 32B) auf den beiden parallelen oberen Schenkelabschnitten (11', 11'; 11B", 11B") gleiten, und dass die Verbindungsabschnitte (32, 32; 32B, 32B) so geformt und auf den parallelen oberen Schenkelabschnitten (11', 11'; 11B", 11B") angeordnet sind, dass im unbelasteten Zustand das mittels der Verbindungsabschnitte (32, 32; 32B, 32B) montierte Federelement (30; 30B) derart hängt, dass ein unterer Zwischenabschnitt (31'), der die parallelen Schenkel (31, 31) des Federelements (30; 30B) verbindet, näher am hinteren Abschnitt (15) der Halteeinheit (10; 10B) liegt als die Verbindungsabschnitte (32, 32; 32B, 32B), wohingegen im belasteten Zustand, beispielsweise mit einer Packung zwischen dem hinteren Abschnitt (15) der Halteeinheit (10; 10B) und dem Federelement (30; 30B), die Betätigungselemente (33, 33; 33B, 33B) in Richtung des hinteren Abschnitts (15) der Halteeinheit (10; 10B) geschoben werden, wodurch das Federelement (30; 30B) dazu gebracht wird, eine vertikale Position parallel zum hinteren Abschnitt (15) der Halteeinheit (10; 10B) in einer Position zur Aufnahme einer Packung einzunehmen, durch eine Kombination aus einer Reibungs- und einer Torsionskraft, die zwischen den Verbindungsabschnitten (32, 32; 32B, 32B) und den parallelen oberen Schenkelabschnitten (11', 11'; 11B", 11B") ausgeübt wird, sodass das Federelement (30; 30B) eine Kraft entlang der parallelen Schenkel (31, 31) ausübt, die es ermöglicht, dass beispielsweise eine Packung (50) zwischen dem Federelement (30; 30B) und dem hinteren Abschnitt der Halteeinheit (10; 10B) eingespannt wird.

2. Halteeinheit (10; 10B) nach Anspruch 1,
**dadurch gekennzeichnet,**
**dass** das oder jedes Federelement (30, 30; 30; 30B, ..., 30B) fest oder lösbar mit den beiden parallelen oberen Schenkelabschnitten (11', 11'; 11B", ..., 11B") verbindbar ist, wobei die beiden Betätigungselemente (33, 33) und die beiden Verbindungsabschnitte (32, 32) an den Enden des Schenkelabschnitts (31, 31) gegenüber dem Zwischenabschnitt (31') angeordnet sind, dass die Betätigungselemente (33, 33) eines Federelements (30, 30; 30; 30B, ..., 30B) in entgegengesetzte Richtungen von der Halteeinheit (10, 10; 10B, ..., 10B) weg nach außen spreizen, wobei jedes ein nach außen vorstehendes gebogenes Drahtteil umfasst, das in derselben Ebene wie eine durch die Federelement Schenkelabschnitte (31, 31) und den Zwischenabschnitt (31') gebildete Ebene verläuft, wobei ein oberer Schenkel, der einen dritten Betätigungselementabschnitt (33‴) an einem inneren, oberen Ende jedes dritten Betätigungselements bildet, eine Biegung von im Wesentlichen 90° bezüglich des jeweiligen Federelement Schenkelabschnitts (31, 31) senkrecht zu der durch die Drahtstruktur des Federelements (30, 30; 30; 30B, ..., 30B) gebildeten Ebene ausbildet, um den Verbindungsabschnitt (32, 32) zu bilden, der eine U Form mit einem freien Schenkel (32", 32") aufweist, der etwas kürzer ist als ein paralleler, innerer Schenkel (32', 32') des U förmigen Verbindungsabschnitts (32, 32), der mit dem dritten Betätigungselementabschnitt (33‴, 33‴) verbunden ist, wobei der U förmige Verbindungsabschnitt (32, 32) so angeordnet ist, dass eine durch die parallelen Schenkel (32', 32") des Verbindungsabschnitts (32, 32) gebildete Ebene einen Winkel zwischen jeweils +/-30-45° mit einer vertikalen Ebene bildet, die senkrecht zu der durch die beiden Federelement Schenkelabschnitte (31, 31) und den Zwischenabschnitt (31') gebildeten Ebene steht.

3. Halteeinheit (10; 10B) nach einem der Ansprüche 1 oder 2,
**dadurch gekennzeichnet,**
**dass** das Federelement (30, 30; 30B, ..., 30B) ein Drahtelement aus Metall, beispielsweise aus Edelstahl oder Federstahl oder aus einem anderen Material, umfasst.

4. Halteeinheit (10; 10B) nach einem der Ansprüche 1 bis 3,
**dadurch gekennzeichnet,**
**dass** die drahtförmige Gestellstruktur (20, 20; 20B, ..., 20B) oder zumindest die oberen Rahmenelementabschnitte (11, 11; 11B, ..., 11B) aus drahtförmigem Metall, z. B. Edelstahl, gefertigt sind, und dass der Durchmesser des das Federelement (30, 30; 30B, ..., 30B) bildenden Drahtelements kleiner ist als der Durchmesser des die Gestellstruktur (20, 20; 20B, ..., 20B) oder zumindest die oberen Rahmenelementabschnitte (11, 11; 11B, ..., 11B) bildenden Drahts.

5. Halteeinheit (10; 10B) nach einem der Ansprüche 1 bis 4,
**dadurch gekennzeichnet,**
**dass** das Federelement (30, 30; 30B, ..., 30B) so auf den beiden parallelen oberen Schenkelabschnitten (11', 11'; 11B", ..., 11B") angeordnet ist, dass jeder parallele obere Schenkelabschnitt (11', 11'; 11B", ..., 11B") zwischen den Schenkeln (32', 32") des Verbindungsabschnitts (32, 32) aufgenommen wird, eine Oberseite eines oberen Betätigungselementabschnitts (33‴) benachbart der Biegung, an der er in den inneren Schenkel (32') des Verbindungsabschnitts (32, 32) übergeht, und eine Unterseite eines Zwischenabschnitts (32‴) des Verbindungsabschnitts (32) mit einem zentralen Abschnitt des inneren Schenkels (32') des Verbindungsabschnitts (32, 32) sowie der Oberseite des oberen Betätigungselementabschnitts (33‴) benachbart der Biegung und der Unterseite des Zwischenabschnitts (32‴) des Verbindungsabschnitts (32) in Kontakt steht.

6. Halteeinheit (10) nach einem der Ansprüche 1 bis 5,
**dadurch gekennzeichnet,**
**dass** die Drahtgestellstruktur (20, 20) einen unteren Rahmenelementabschnitt (12) umfasst, dass der untere Rahmenelementabschnitt (12) eine im Wesentlichen U förmige Gestalt mit zwei parallelen unteren Schenkelabschnitten (12', 12') und einem vorderen Schenkel Verbindungsabschnitt (12") aufweist, der im montierten Zustand in einer horizontalen Ebene senkrecht zur Montageschienenanordnung (40) in einer Ebene parallel zum oberen Rahmenelementabschnitt (11) anordenbar ist und als Auflage für eine zu haltende Packung dient, dass die Drahtgestellstruktur (20, 20) ferner einen hinteren Abschnitt (15) umfasst, der zwei parallel angeordnete obere vertikale Zweige (15', 15') aufweist, die mittels einer oberen und einer unteren Verbindungsstange (18', 18) miteinander verbunden sind, die parallel in einem Abstand voneinander angeordnet sind und die beispielsweise auf einer Rückseite der oberen vertikalen Zweige (15', 15') aufgelötet oder aufgeschweißt sind und geringfügig davon vorstehen, wodurch ein Verbindungsabschnitt (19) zur Verbindung mit der Montageschienenanordnung (40; 40A) gebildet wird.

7. Haltesystem (100; 101) für Packungen für Produkte, beispielsweise Wegwerfartikel, umfassend eine Montageschienenanordnung (40; 40A; 40B) zur vertikalen Befestigung an einer vertikalen Fläche,
**dadurch gekennzeichnet,**
**dass** es eine oder mehrere Halteeinheiten (10, 10; 10B, ..., 10B) nach einem der Ansprüche 1 bis 6 umfasst, die fest oder lösbar an der Montageschienenanordnung (40; 40A; 40B) montiert sind, wobei die beiden parallelen oberen Schenkelabschnitte (11', 11'; 11B", ..., 11B") der oder jeder Halteeinheit (10, 10; 10B, ..., 10B) im Wesentlichen horizontal und senkrecht von der Montageschienenanordnung (40; 40A; 40B) abstehen, wobei das oder die Federelemente (30, 30; 30; 30B, ..., 30B) der oder jeder Halteeinheit (10; 10B) zwischen einer hinteren Position nahe einer vertikalen Erstreckungsebene der Montageschienenanordnung (40; 40A; 40B) und der vorderen Position nahe äußeren Endabschnitten der parallelen oberen Schenkelabschnitte (11', 11'; 11B", ..., 11B") bewegbar sind, und wobei im unbelasteten Zustand das Federelement (30, 30; 30; 30B, ..., 30B) der oder jeder Halteeinheit (10; 10B) derart hängt, dass der untere Zwischenabschnitt (31), der die parallelen Schenkel (31, 31) des Federelements (30, 30; 30; 30B, ..., 30B) verbindet, näher an der vertikalen Erstreckungsebene der Montageschienenanordnung (40; 40A; 40B) liegt als die Verbindungsabschnitte (32, 32; 32B, ..., 32B), wohingegen im belasteten Zustand das oder die Federelemente (30, 30; 30; 30B, ..., 30B) durch die genannte Kombination aus einer Reibungs- und einer Torsionskraft, die zwischen den Verbindungsabschnitten (32, 32; 32B, ..., 32B) und den parallelen oberen Schenkelabschnitten (11', 11'; 11B", ..., 11B") ausgeübt wird, in Richtung der vertikalen Erstreckungsebene der Montageschienenanordnung (40; 40A; 40B) gedrängt werden, sodass beispielsweise eine Packung (50) zwischen dem Federelement (30, 30; 30; 30B, ..., 30B) einer jeweiligen Halteeinheit (10, 10; 10B, ..., 10B) und der Montageschienenanordnung (40; 40A; 40B) oder dem hinteren Abschnitt der jeweiligen Halteeinheit (10, 10; 10B, ..., 10B) eingespannt werden kann.

8. Haltesystem (100) nach Anspruch 7,
**dadurch gekennzeichnet,**
**dass** die Montageschienenanordnung eine Montageschiene (40; 40A) umfasst, die interne Haltenuten oder Aufnahmeräume (41A, 41A, 41A', 41A', 41A", 41A") aufweist, die paarweise und senkrecht zur Längserstreckung der Montageschiene (40; 40A) angeordnet sind, wobei jedes Paar interner Haltenuten oder Aufnahmeräume (41A, 41A, 41A', 41A', 41A", 41A") die Aufnahme einer Halteeinheit (10, 10) durch Aufnahme der oberen und unteren Verbindungsstangen (18', 18) an einem hinteren Abschnitt der drahtförmigen Gestellstruktur (20, 20) durch jeweilige Aufnahmeöffnungen (42A, 42A, 42A', 42A', 42A", 42A") an einer Vorderseite der Montageschiene (40; 40A) ermöglicht, wobei die Montageschiene (40; 40A) ferner Befestigungselemente oder Befestigungslöcher (44, 44'; 44A, 44A', 44A") oder dergleichen zur Wandbefestigung aufweist.

9. Haltesystem (100) nach Anspruch 8,
**dadurch gekennzeichnet,**
**dass** jede Haltenut oder jeder Aufnahmeraum (41A, 41A, 41A', 41A', 41A", 41A") eine Höhe (h) aufweist, die größer ist als eine Höhe (h1) einer entsprechenden Aufnahmeöffnung (42A, 42A, 42A', 42A', 42A", 42A"), und dass ein unterer Endabschnitt der internen Haltenut oder des Aufnahmeraums (41A, 41A, 41A', 41A', 41A", 41A") unterhalb einer Unterkante der entsprechenden Aufnahmeöffnung (42A, 42A, 42A', 42A', 42A", 42A") liegt, wenn sich die Montageschiene (40; 40A) in vertikal angeordnetem Zustand befindet, wodurch eine montierte Halteeinheit in ihrer Position gehalten wird, indem die oberen und unteren Verbindungsstangen (18', 18) innerhalb der internen Haltenut oder des Aufnahmeraums (41A, 41A, 41A', 41A', 41A", 41A") aufgenommen werden und dabei mit deren Bodenabschnitten in Kontakt stehen.

10. Haltesystem (100) nach einem der Ansprüche 7 bis 9,
**dadurch gekennzeichnet,**
**dass** die oder jede Halteeinheit (10, 10) lösbar an der Montageschiene (40) montierbar ist.

11. Haltesystem (100) nach einem der Ansprüche 7 bis 10,
**dadurch gekennzeichnet,**
**dass** die Montageschiene (40) aus Al, insbesondere aus eloxiertem Al, gefertigt ist.

12. Haltesystem (101) nach Anspruch 7,
**dadurch gekennzeichnet,**
**dass** die Montageschienenanordnung (40B) zwei parallele, im montierten Zustand der Montageschienenanordnung (40B) vertikal verlaufende Zweige (45, 45) umfasst, die mittels mindestens eines oberen und eines unteren Verbindungselements (46, 46', 46") zur Befestigung an einer Wand oder dergleichen miteinander verbunden sind, wobei eine Anzahl von Halteeinheiten (10B, 10B, 10B, 10B) vertikal übereinander angeordnet sind, wobei jede Halteeinheit (10B, 10B, 10B, 10B) fest, beispielsweise durch Schweißen oder Löten, an den vertikal verlaufenden Zweigen (45, 45) der Montageschienenanordnung (40B) befestigt ist, wobei jede Halteeinheit (10B, 10B, 10B, 10B) einen oberen Rahmenelementabschnitt (11B, 11B, 11B, 11B) umfasst, der zwei parallele, einen oberen und einen unteren, U förmige Zweigabschnitte (11B', 11B") aufweist, die von jedem der beiden parallelen vertikalen Zweige (45, 45) senkrecht dazu in Vorwärtsrichtung abstehen, wobei die oberen Zweigabschnitte (11B", 11B") mittels eines dazwischenliegenden hinteren Zweigs (11B‴) miteinander verbunden sind, wobei der dazwischenliegende hintere Zweig (11B‴) senkrecht zu den beiden parallelen vertikal verlaufenden Zweigen (45, 45) verläuft und in einer hinteren Ebene auf diese geschweißt oder gelötet ist, wobei die oberen Zweigabschnitte (11B', 11B') einer Halteeinheit (10B), über der eine weitere Halteeinheit angeordnet ist, als Auflageebene, beispielsweise für eine Packung, der darüber angeordneten Halteeinheit dienen, und wobei das Federelement (30B) an den unteren Zweigabschnitten (11B", 11B") angeordnet ist, wobei jeder obere Rahmenelementabschnitt (11B) aus einer gebogenen Drahtstruktur gefertigt ist.

## Revendications

1. Unité de maintien (10 ; 10B), comprenant une structure de cadre en fil (20 ; 20B) et un élément ressort (30 ; 30B) réalisé en fil, adaptée pour recevoir et retenir un emballage (50) de manière à permettre l'accès aux produits ou articles contenus dans l'emballage (50),
**caractérisée en**
**ce qu'**elle comprend une section d'élément de cadre supérieur (11 ; 11B) comprenant deux portions de jambe supérieures parallèles (11', 11' ; 11B", 11B") faisant saillie sensiblement horizontalement et perpendiculairement depuis une section arrière (15) de l'unité de maintien, ce que l'élément ressort (30 ; 30B) est sensiblement en forme de jambe en U et comprend un élément d'actionnement (33, 33 ; 33B, 33B) et une portion de connexion (32, 32 ; 32B, 32B) aux deux extrémités des jambes parallèles (31, 31) dudit élément ressort (30 ; 30B), ce que les portions de connexion (32, 32 ; 32B, 32B) de l'élément ressort (30 ; 30B) sont montées de manière coulissante sur les portions de jambe supérieures parallèles (11', 11' ; 11B", 11B") de telle sorte que l'élément ressort (30 ; 30B) peut être déplacé entre une position arrière proche de la section arrière (15) de l'unité de maintien (10 ; 10B) et une position avant adjacente aux portions d'extrémité extérieures des portions de jambe supérieures parallèles (11', 11' ; 11B", 11B") par le coulissement des portions de connexion (32, 32 ; 32B, 32B) sur les deux portions de jambe supérieures parallèles (11', 11' ; 11B", 11B") grâce à l'application simultanée d'une force de poussée/tirage sur chaque élément d'actionnement (33, 33 ; 33B, 33B), et **en ce que** les portions de connexion (32, 32 ; 32B, 32B) sont conformées et disposées sur les portions de jambe supérieures parallèles (11', 11' ; 11B", 11B") de telle sorte que, dans un état non chargé, l'élément ressort (30 ; 30B) monté au moyen des portions de connexion (32, 32 ; 32B, 32B) pendra de manière à ce qu'une section intermédiaire inférieure (31') reliant les jambes parallèles (31, 31) dudit élément ressort (30 ; 30B) soit située plus près de la section arrière (15) de l'unité de maintien (10 ; 10B) que les portions de connexion (32, 32 ; 32B, 32B), tandis que dans un état chargé, par exemple avec un emballage disposé entre la section arrière (15) de l'unité de maintien (10 ; 10B) et l'élément ressort (30 ; 30B), les éléments d'actionnement (33, 33 ; 33B, 33B) sont poussés vers la section arrière (15) de l'unité de maintien (10 ; 10B), contraignant l'élément ressort (30 ; 30B) à adopter une position verticale parallèle à la section arrière (15) de l'unité de maintien (10 ; 10B) dans une position pour recevoir un emballage, grâce à une combinaison d'une force de frottement et d'une force de torsion exercée entre les portions de connexion (32, 32 ; 32B, 32B) et les portions de jambe supérieures parallèles (11', 11' ; 11B", 11B"), de telle sorte que l'élément ressort (30 ; 30B) appliquera une force le long des jambes parallèles (31, 31) permettant par exemple à un emballage (50) d'être serré entre l'élément ressort (30 ; 30B) et la section arrière de l'unité de maintien (10 ; 10B).

2. Unité de maintien (10 ; 10B) selon la revendication 1,
**caractérisée en**
**ce que** le, ou chaque, élément ressort (30, 30 ; 30 ; 30B, ..., 30B) est adapté pour être connecté de manière fixe ou détachable aux deux portions de jambe supérieures parallèles (11', 11' ; 11B", .., 11B"), les deux éléments d'actionnement (33, 33) et les deux portions de connexion (32, 32) étant disposés aux extrémités de la section de jambe (31, 31) opposées à la section intermédiaire (31'), ce que les éléments d'actionnement (33, 33) d'un élément ressort (30, 30 ; 30 ; 30B, ..., 30B) s'évasent vers l'extérieur dans des directions opposées en s'éloignant de l'unité de maintien (10, 10 ; 10B, ..., 10B) dans des directions opposées, chacun comprenant une portion de fil coudée faisant saillie vers l'extérieur s'étendant dans le même plan qu'un plan formé par les sections de jambe d'élément ressort (31, 31) et la section intermédiaire (31'), dans lequel une jambe supérieure formant une troisième section d'élément d'actionnement (33‴) à une extrémité intérieure supérieure de chaque troisième élément d'actionnement forme un coude d'environ 90° par rapport à la section de jambe d'élément ressort respective (31, 31), perpendiculairement au plan formé par la structure en fil de l'élément ressort (30, 30 ; 30 ; 30B, ..., 30B), pour former ladite portion de connexion (32, 32) ayant une forme de U avec une jambe libre (32", 32") quelque peu plus courte qu'une jambe intérieure parallèle (32', 32') de la portion de connexion en forme de U (32, 32) se connectant à la troisième section d'élément d'actionnement (33‴, 33‴), ladite portion de connexion en forme de U (32, 32) étant ainsi disposée qu'un plan formé par les jambes parallèles (32', 32") de la portion de connexion (32, 32) forme un angle compris entre +/-30-45° respectivement avec un plan vertical perpendiculaire au plan formé par les deux sections de jambe d'élément ressort (31, 31) et la section intermédiaire (31').

3. Unité de maintien (10 ; 10B) selon l'une quelconque des revendications 1 ou 2,
**caractérisée en**
**ce que** l'élément ressort (30, 30 ; 30B, .., 30B) comprend un élément en fil de métal, par exemple en acier inoxydable ou en acier à ressort ou en un autre matériau.

4. Unité de maintien (10 ; 10B) selon l'une quelconque des revendications 1 à 3,
**caractérisée en**
**ce que** la structure de cadre en forme de fil (20, 20 ; 20B, ..., 20B), ou du moins les sections d'élément de cadre supérieur (11, 11 ; 11B, .., 11B), est/sont réalisée(s) en métal en forme de fil, par exemple en acier inoxydable, et **en ce que** le diamètre de l'élément en fil formant l'élément ressort (30, 30 ; 30B, .., 30B) est inférieur au diamètre du fil formant la structure de cadre (20, 20 ; 20B, ..., 20B) ou du moins les sections d'élément de cadre supérieur (11, 11 ; 11B, .., 11B).

5. Unité de maintien (10 ; 10B) selon l'une quelconque des revendications 1 à 4,
**caractérisée en**
**ce que** l'élément ressort (30, 30 ; 30B, .., 30B) est adapté pour être disposé sur les deux portions de jambe supérieures parallèles (11', 11' ; 11B", .., 11B") de telle sorte que chaque portion de jambe supérieure parallèle (11', 11' ; 11B", .., 11B") est reçue entre les jambes (32', 32") de la portion de connexion (32, 32), un côté supérieur d'une section d'élément d'actionnement supérieur (33‴) adjacent au coude où il se transforme en la jambe intérieure (32') de la portion de connexion (32, 32) et une portion intermédiaire (32‴) de côté inférieur de la portion de connexion (32), et est en contact avec une portion centrale de la jambe intérieure (32') de la portion de connexion (32, 32) et le côté supérieur de la section d'élément d'actionnement supérieur (33‴) adjacent au coude et le côté inférieur de la portion intermédiaire (32‴) de la portion de connexion (32).

6. Unité de maintien (10) selon l'une quelconque des revendications 1 à 5,
**caractérisée en**
**ce que** la structure de cadre en fil (20, 20) comprend une section d'élément de cadre inférieur (12), ce que la section d'élément de cadre inférieur (12) a une forme sensiblement en U avec deux portions de jambe inférieures parallèles (12', 12') et une portion de liaison de jambe avant (12") disposée pour être placée dans un plan horizontal perpendiculairement au dispositif de rail de montage (40) dans un état monté dans un plan parallèle à la section d'élément de cadre supérieur (11) et servant de support pour un emballage à maintenir, ce que la structure de cadre en fil (20, 20) comprend en outre une section arrière (15) comprenant deux branches verticales supérieures disposées parallèlement (15', 15') reliées entre elles au moyen d'une tige de liaison supérieure et d'une tige de liaison inférieure (18', 18) qui sont disposées parallèlement à distance l'une de l'autre et qui sont par exemple soudées ou brasées sur les branches verticales supérieures (15', 15') sur un côté arrière de celles-ci et font légèrement saillie à partir de celles-ci formant une section de connexion (19) pour la connexion au dispositif de rail de montage (40 ; 40A).

7. Système de maintien (100 ; 101) pour des emballages de produits, par exemple des articles jetables, comprenant un dispositif de rail de montage (40 ; 40A ; 40B) pour un montage vertical sur une surface verticale,
**caractérisé en**
**ce qu'**il comprend une ou plusieurs unités de maintien (10, 10 ; 10B, ..., 10B) selon l'une quelconque des revendications 1 à 6 fixées ou montées de manière détachable sur le dispositif de rail de montage (40 ; 40A ; 40B),
dans lequel les deux portions de jambe supérieures parallèles (11', 11' ; 11B", .., 11B") de la, ou de chaque, unité de maintien (10, 10 ; 10B, ..., 10B) font saillie sensiblement horizontalement et perpendiculairement depuis le dispositif de rail de montage (40 ; 40A ; 40B), dans lequel le ou les éléments ressort (30, 30 ; 30 ; 30B, ..., 30B) de la, ou de chaque, unité de maintien (10 ; 10B) peuvent être déplacés entre une position arrière proche d'un plan vertical d'extension du dispositif de rail de montage (40 ; 40A ; 40B) et la position avant adjacente aux portions d'extrémité extérieures des portions de jambe supérieures parallèles (11', 11' ; 11B", .., 11B"), et, dans l'état non chargé, le ou les éléments ressort (30, 30 ; 30 ; 30B, ..., 30B) de la, ou de chaque, unité de maintien (10 ; 10B) pendront de telle sorte que la section intermédiaire inférieure (31) reliant les jambes parallèles (31, 31) dudit élément ressort (30, 30 ; 30 ; 30B, ..., 30B) sera située plus près du plan vertical d'extension du dispositif de rail de montage (40 ; 40A ; 40B) que les portions de connexion (32, 32 ; 32B, .., 32B), tandis que dans l'état chargé, le ou les éléments ressort (30, 30 ; 30 ; 30B, ..., 30B) sont contraints vers le plan vertical d'extension du dispositif de rail de montage (40 ; 40A ; 40B) grâce à ladite combinaison d'une force de frottement et d'une force de torsion exercée entre les portions de connexion (32, 32 ; 32B, .., 32B) et les portions de jambe supérieures parallèles (11', 11' ; 11B", .., 11B"), permettant par exemple à un emballage (50) d'être serré entre l'élément ressort (30, 30 ; 30 ; 30B, ..., 30B) d'une unité de maintien respective (10, 10 ; 10B, ..., 10B) et le dispositif de rail de montage (40 ; 40A ; 40B) ou la section arrière de l'unité de maintien respective (10, 10 ; 10B, ..., 10B).

8. Système de maintien (100) selon la revendication 7,
**caractérisé en**
**ce que** le dispositif de rail de montage comprend un rail de montage (40 ; 40A) qui comprend des rainures de maintien internes ou des espaces d'accueil (41A, 41A, 41A', 41A', 41A", 41A") qui sont disposés par paires et perpendiculairement à l'extension longitudinale du rail de montage (40 ; 40A), chaque paire de rainures de maintien internes ou d'espaces d'accueil (41A, 41A, 41A', 41A', 41A", 41A") permettant la réception d'une unité de maintien (10, 10) par la réception des tiges de liaison supérieure et inférieure (18', 18) au niveau d'une section arrière de la structure de cadre en forme de fil (20, 20) par l'intermédiaire d'ouvertures de réception respectives (42A, 42A, 42A', 42A', 42A", 42A") sur un côté avant du rail de montage (40 ; 40A), le rail de montage (40 ; 40A) comprenant en outre des éléments de montage ou des trous de montage (44, 44' ; 44A, 44A', 44A") ou similaires pour le montage sur un mur.

9. Système de maintien (100) selon la revendication 8,
**caractérisé en**
**ce que** chaque rainure de maintien ou espace d'accueil (41A, 41A, 41A', 41A', 41A", 41A") a une hauteur (h) supérieure à une hauteur (h1) d'une ouverture de réception correspondante (42A, 42A, 42A', 42A', 42A", 42A") et **en ce qu'**une portion d'extrémité inférieure de la rainure de maintien interne ou espace d'accueil (41A, 41A, 41A', 41A', 41A", 41A") est située en dessous d'un bord inférieur de l'ouverture de réception correspondante (42A, 42A, 42A', 42A', 42A", 42A") dans un état disposé verticalement du rail de montage (40 ; 40A) permettant à une unité de maintien montée d'être maintenue en place grâce aux tiges de liaison supérieure et inférieure (18', 18) étant reçues à l'intérieur de la rainure de maintien interne ou espace d'accueil (41A, 41A, 41A', 41A', 41A", 41A") en contact avec des portions inférieures de ceux-ci.

10. Système de maintien (100) selon l'une quelconque des revendications 7 à 9,
**caractérisé en**
**ce que** la ou chaque unité de maintien (10, 10) peut être montée de manière détachable sur le rail de montage (40).

11. Système de maintien (100) selon l'une quelconque des revendications 7 à 10,
**caractérisé en**
**ce que** le rail de montage (40) est réalisé en Al, plus particulièrement en Al anodisé.

12. Système de maintien (101) selon la revendication 7,
**caractérisé en**
**ce que** le dispositif de rail de montage (40B) comprend deux branches parallèles (45, 45) s'étendant verticalement dans un état monté du dispositif de rail de montage (40B) qui sont reliées entre elles au moyen d'au moins un élément de connexion supérieur et un élément de connexion inférieur (46, 46', 46") pour le montage sur un mur ou similaire, un certain nombre d'unités de maintien (10B, 10B, 10B, 10B) disposées verticalement l'une au-dessus de l'autre, chaque unité de maintien (10B, 10B, 10B, 10B) étant montée de manière fixe, par exemple par soudage ou brasage, sur les branches s'étendant verticalement (45, 45) du dispositif de rail de montage (40B), chaque unité de maintien (10B, 10B, 10B, 10B) comprenant une section d'élément de cadre supérieur (11B, 11B, 11B, 11B) comprenant deux portions de branche en forme de U parallèles, une supérieure et une inférieure (11B', 11B") faisant saillie depuis, et perpendiculairement à, chacune desdites deux branches verticales parallèles (45, 45) dans une direction avant, lesdites portions de branche supérieures (11B", 11B") étant reliées entre elles au moyen d'une branche arrière intermédiaire (11B‴), la branche arrière intermédiaire (11B‴) s'étendant perpendiculairement à, et étant soudée ou brasée sur, les deux branches s'étendant verticalement parallèles (45, 45) dans un plan arrière, les portions de branche supérieures (11B', 11B') d'une unité de maintien (10B), ayant une unité de maintien disposée au-dessus d'elle, servant de plan de support, par exemple pour un emballage, de l'unité de maintien disposée au-dessus de celle-ci, et l'élément ressort (30B) étant disposé sur les portions de branche inférieures (11B", 11B"), chaque section d'élément de cadre supérieur (11B) étant réalisée en une structure en fil coudée.
